# EUROPÄISCHE PATENTSCHRIFT

(11) **EP 0 364 926 B1**
(45) Veröffentlichungstag und Bekanntmachung des Hinweises auf die Patenterteilung: **16.02.1994**
(21) Anmeldenummer: 89119181.9
(22) Anmeldetag: 16.10.1989
(51) Int. Cl.: C12N 15/13, C12N 15/58, C07K 3/00, C07K 3/12, C07K 3/28

(54) **Aktivierung von gentechnologisch hergestellten, in Prokaryonten exprimierten Antikörpern**
Activation of recombinant antibodies expressed in prokaryotes
Activation des anticorps recombinants exprimés chez les procaryotes

(30) Priorität: 17.10.1988 DE 3835350
(43) Veröffentlichungstag der Anmeldung: 25.04.1990
(73) Patentinhaber: BOEHRINGER MANNHEIM GMBH, 68298 Mannheim (DE)
(72) Erfinder: Rudolph, Rainer, Dr.rer.nat., D-8120 Weilheim (DE); Buchner, Johannes, D-8400 Regensburg (DE); Lenz, Helmut, Dr.rer.nat., D-8132 Tutzing (DE)
(74) Vertreter: Huber, Bernhard, Dipl.-Chem.

(56) Entgegenhaltungen:
- EP-A- 0 114 506
- EP-A- 0 192 629
- EP-A- 0 211 592
- EP-A- 0 219 874
- EP-A- 0 236 209
- EP-A- 0 241 022
- WO-A-87/02985
- GB-A- 2 176 702
- THE JOURNAL OF BIOLOGICAL CHEMISTRY Band 241, Nr. 22, 25.November 1966, Seiten 5225-5232, Baltimore, US; M.H. FREEDMAN et al.: "Recoveryof Specific Activity upon Reoxidation of Completely Reduced Polyalanyl RabbitAntibody"
- CHEMICAL ABSTRACTS Band 113, Nr. 3, 16. Juli1990, Seite 480, Zusammenfassung Nr. 21920s, Columbus, Ohio, US; J. BUCHNER etal.: "Renaturation of antibodies after expression in E. coli."& DECHEMA Biotechnol. Conf. 1989, Band 3, Seiten 1035-1040

## Beschreibung

Die Erfindung betrifft ein Verfahren zur Aktivierung von gentechnologisch hergestellten, in Prokaryonten exprimierten biologisch aktiven Proteinen.

Die gentechnologische Herstellung von Proteinen, wie z.B. Antikörpern in heterologen Wirtsorganismen, führt zur Bildung inaktiver, schwer löslicher Proteinaggregate, sogenannten "inclusion bodies". Es wird vermutet, daß die Bildung solcher "inclusion bodies" unter anderem eine Folge der bei der Expression entstehenden hohen Proteinkonzentrationen in der Zelle ist. Um biologisch aktive Proteine zu erhalten, müssen die inclusion bodies durch Denaturierung und Reduktion aufgelöst werden, und sodann die dreidimensionale Struktur des Proteins in ihrer nativen Raumform durch Einstellung geeigneter Lösungsbedingungen wieder hergestellt werden (M. Sela et al., 1957, Science 125, 691). Die vollständige Entfaltung wird durch Zusatz hoher Konzentrationen chaotroper Agentien wie Harnstoff und Guanidin erreicht (R. Jaenicke, (1987) Prog. Biophys. Molec. Biol. 49, 117-237). Zur Reduktion von Disulfidbrücken werden starke Reduktionsmittel wie ß-Mercaptoethanol oder 1,4-Dithioerithrit verwendet.

Bei der Renaturierung aus dem denaturierten Zustand treten jedoch zwei Konkurrenzreaktionen auf. Neben der gewünschten Faltung in den nativen Zustand beobachtet man Aggregatbildung (G. Zettlmeißl et al., Biochemistry 18, 5567). Um das Gleichgewicht auf die Seite des nativen Moleküls zu verlagern, wählt man Bedingungen, die zum einen die Etablierung falsch gefalteter und damit instabilerer Moleküle und deren unspezifische Wechselwirkung zu Aggregaten verhindern, zum anderen aber die Rückfaltung in den nativen Zustand nicht behindern. Man erreicht dies durch Zusatz chaotroper Agentien in labilisierenden Konzentrationen. Zusätzlich muß beachtet werden, daß sich die Proteinkonzentration kritisch auf die Renaturierungsausbeute auswirkt (EP 0 241 022). Bei im nativen Zustand disulfidverbrückten Proteinen gilt es zusätzlich während der Renaturierung Redoxbedingungen zu schaffen, die es ermöglichen, falsch verbrückte Cystine wieder zu reduzieren und richtig gepaarte zu populieren. Sogenannte "Oxidoshuffling solutions" aus reduziertem und oxidiertem Thiolreagens erhöhen die Ausbeute an nativ strukturiertem und disulfidverbrücktem Protein.

Daß die Renaturierung von Antikörpern nach vollständiger Denaturierung und Reduktion möglich ist, wurde von Haber erstmals am Beispiel eines Fab-Fragmentes gezeigt (E. Haber, Biochemistry 52, 1099-1106 (1964). Die Ausbeuten betrugen 12 bis 14 %. Bestätigt wurden diese durch Ergebnisse zur Renaturierung eines Fab-Fragmentes von Whitney und Tanford. Sie erzielten Ausbeuten von 8 % (P.L. Whitney, C. Tanford, Proc. Natl. Acad. Sci. USA 53, 524 (1965)). Ein vollständiger Antikörper wurde erstmals von Freedman und Sela erfolgreich renaturiert. Die Ausbeuten betrugen zwischen 20 und 25 % (M.H. Freedman, M. Sela, J. Biol. Chem. 241, 2383-2396 (1966), J. Biol. Chem. 241, 5225-5232 (1966)). Anzumerken ist, daß das native Ausgangsmolekül zur Verbesserung der Lösungseigenschaften polyalanyliert wurde.

Bei den angeführten Arbeiten gilt es zu beachten, daß es sich um polyklonale Antikörper bzw. Antikörper-Fragmente handelt, also ein Gemisch von Antikörpern mit unterschiedlichen Paratopen und unterschiedlichen Affinitätskonstanten.

Da die schweren und leichten Ketten dieser heterologen Population bei der Renaturierung rein statistisch assoziieren, werden hier renaturierte Moleküle erzeugt, die mit den nativen bezüglich Bindungsspezifität und Affinität nicht übereinstimmen.

Wie bei anderen eukaryontischen Proteinen, die in E. coli kloniert und exprimiert wurden, fielen auch in E. coli exprimierte schwere und leichte Ketten von Antikörpern in Form unlöslicher "inclusion bodies" an (S. Cabilly et al., Proc. Natl. Acad. Sci. USA 81, 3273-3277 (1984), M.Y. Boss et al., 1984, Nucleic Acids Research 12, 3791-3806). Für Antikörper aus transformierten Mikroorganismen wurden von Cabilly et al. und Boss et al. Verfahren beschrieben, die die Renaturierung funktioneller Antikörper ermöglichen sollen. Diese Verfahren lieferten jedoch für monoklonale Antikörper nur Ausbeuten zwischen ca. 0,2 % und 5 %.

Der Erfindung liegt daher die Aufgabe zugrunde, ein Verfahren zur Reaktivierung von in Prokaryonten exprimierten biologisch aktiven Proteinen bereitzustellen, wobei die in Form von "inclusion bodies" nach Expression erhaltenen Proteine in guter Ausbeute in ihre aktive renaturierte Form überführt werden können.

Gelöst wird diese Aufgabe durch ein Verfahren zur Aktivierung von gentechnologisch hergestellten, in Prokaryonten exprimierten biologisch aktiven Proteinen nach Zellaufschluß durch Solubilisierung unter denaturierenden und reduzierenden Bedingungen und nachfolgende Reaktivierung unter oxidierenden und renaturierenden Bedingungen, das dadurch gekennzeichnet ist, daß man bei einer Proteinkonzentration von 1 bis 1000 µg/ml arbeitet und zwischen der Solubilisierung und der Reaktivierung eine Dialyse gegen einen Puffer mit einem pH-Wert zwischen 1 und 4, enthaltend 4 bis 8 mol/l Guanidinhydrochlorid oder 6 bis 10 mol/l Harnstoff, durchführt.

Überraschenderweise wurde nämlich festgestellt, daß nach einer Dialyse in Gegenwart von Harnstoff oder insbesondere Guanidinhydrochlorid außerordentlich erhöhte Ausbeuten an aktivem Protein nach der Reaktivierung erhalten werden.

Die Erfindung eignet sich zur Anwendung bei allen gentechnologisch in Prokaryonten hergestellten biologisch aktiven Proteine, vorzugsweise für Antikörper und deren Fragmente und für t-PA und t-PA-ähnliche Proteine bzw. -Abkömmlinge.

In einer bevorzugten Ausführungsform der Erfindung enthält der Dialysepuffer 5 bis 7 mol/l Guanidinhydrochlorid.

Bei der Reaktivierung ist es bevorzugt, bei einem pH-Wert von 9 bis 12, einer GSH-Konzentration von 0,1 bis 20 mmol/l, einer GSSG-Konzentration von 0,01 bis 3 mmol/l und mit einer nicht denaturierenden Konzentration eines Denaturierungsmittels zu arbeiten und die Reaktivierung über einen Zeitraum von 1 bis 300 Stunden durchzuführen. Besonders bevorzugt beträgt die GSH-Konzentration hierbei 0,2 bis 10 mmol/l und/oder die GSSG-Konzentration 0,05 bis 1 mmol/l.

In einer anderen bevorzugten Ausführungsform der Erfindung überführt man in der Stufe der Reaktivierung zuerst durch Zugabe von GSSG unter denaturierenden Bedingungen die Thiolgruppen der Antikörper in die gemischten Disulfide von Antikörper und Glutathion, dialysiert erneut gegen den Guanidinhydrochlorid- oder Harnstoff enthaltenden Puffer und reaktiviert dann bei einem pH-Wert von 6 bis 10, einer GSH-Konzentration von 0,1 bis 5 mmol/l und mit einer nicht denaturierenden Konzentration eines Denaturierungsmittels über einen Zeitraum von 1 bis 300 Stunden.

Als Denaturierungsmittel kann in der Regel ein für Aktivierungen unter oxidierenden Bedingungen üblicherweise verwendetes Denaturierungsmittel oder Arginin eingesetzt werden. Bevorzugt verwendet man als Denaturierungsmittel Arginin, Guanidinhydrochlorid und/oder wenigstens eine Verbindung der allgemeinen Formel

R₂-CO-NRR₁ (I),

in der R und R₁ H oder Alkyl mit 1 bis 4 C-Atomen und R₂ H oder NRR₁ oder Alkyl mit 1 bis 3 C-Atomen bedeuten. Diese Denaturierungsmittel können ferner als Gemische verwendet werden. Bevorzugt beträgt die Konzentration an Arginin und/oder Guanidinhydrochlorid 0,1 bis 1,0 mol/l, insbesondere 0,25 bis 0,8 mol/l. Die Konzentration der Verbindung der allgemeinen Formel I beträgt bevorzugt 0,5 bis 4 mol/l, insbesondere 1 bis 3,5 mol/l. In einer weiteren bevorzugten Ausführungsform der Erfindung wird die Reaktivierungsstufe in Gegenwart eines Fremdproteins durchgeführt. Als solches eignet sich in der Regel jedes Fremdprotein, solange es nicht proteolytisch wirksam ist; vorzugsweise wird Rinderserumalbumin (BSA) verwendet, z. B. in einer Menge von 1 bis 3 mg/ml. Der Zusatz von BSA bewirkt eine leichte Erhöhung der Ausbeute und Stabilisierung des Proteins (wahrscheinlich durch Schutz vor Oberflächendenaturierung und/oder proteolytischem Abbau).

Die üblichen Verfahrensbedingungen können den für Reaktivierungsstufen aus dem Stand der Technik bekannten und üblichen Bedingungen entsprechen. Die Reaktivierung wird zweckmäßigerweise bei etwa 5°C bis 30°C durchgeführt, vorzugsweise bei 10°C. Die der Dialyse und Reaktivierungsstufe (Reoxidation/Aktivierung) vorausgehenden und nachfolgenden Verfahrensschritte, wie Zellaufschluß, Solubilisierung (Solubilisierung, Reduktion) können nach aus dem Stand der Technik, z. B. der EP-A-0 114 506, EP-A-0 093 619 für die Reaktivierung von heterolog exprimierten Proteinen bzw. von t-PA bekannten und üblichen Methoden durchgeführt werden; für ein im Hinblick auf Ausbeute und Aktivierung optimales Ergebnis kann es aber zweckmäßig sein, einzelne oder alle Verfahrensschritte unter Berücksichtigung einer oder mehrerer der hier erläuterten Verfahrensausgestaltungen durchzuführen.

Der Zellaufschluß kann durch hierfür übliche Methoden durchgeführt werden, z. B. mittels Ultraschall, Hochdruckdispersion oder Lysozym. Er wird vorzugsweise in einer zur Einstellung eines neutralen bis schwach sauren pH-Wert geeigneten Pufferlösung als Suspensionsmedium durchgeführt, wie z. B. in 0,1 mol/l Tris/HCl. Nach dem Zellaufschluß werden die unlöslichen Bestandteile (inclusion bodies) in beliebiger Weise abgetrennt, vorzugsweise durch Abzentrifugieren oder durch Filtration. Nach dem Waschen mit Agentien, die die Proteine nicht stören, fremde Zellproteine jedoch möglichst lösen, z. B. Wasser, Phosphat-Pufferlösung, gegebenenfalls unter Zusatz milder Detergenzien wie Triton, wird der Niederschlag (Pellet) der Solubilisierung (Solubilisierung/Reduktion) unterworfen.

Die Solubilisierung erfolgt bevorzugt im alkalischen pH-Bereich, insbesondere bei pH 8,6 ± 0,4 und in Gegenwart eines Reduktionsmittels aus der Mercaptangruppe und eines Denaturierungsmittels.

Als Denaturierungsmittel können die für Solubilisierungen aus dem Stand der Technik, z. B. aus der EP-A-0 114 506, bekannten und üblichen Denaturierungsmittel verwendet werden, und insbesondere Guanidinhydrochlorid oder Verbindungen der allgemeinen Formel I. Zweckmäßigerweise geschieht dies in einer Konzentration an Guanidinhydrochlorid von 6 mol/l, bzw. einer Konzentration an der Verbindung der allgemeinen Formel von 8 mol/l.

Als Reduktionsmittel aus der Mercaptangruppe kann z. B. reduziertes Glutation (GSH) oder 2-Mercaptoethanol, z. B. in einer Konzentration von ca. 50 bis 400 mmol/l und/oder insbesondere DTE (Dithioerythritol) bzw. DTT (Dithiothreitol), z. B. in einer Konzentration von ca. 80 bis 400 mmol/l, bzw. Cystein verwendet werden. Die Solubilisierung erfolgt zweckmäßigerweise bei Raumtemperatur während einer Dauer (Inkubation) von 0,5 bis mehreren Stunden, vorzugsweise von 2 Stunden. Zur Verhinderung der Aufoxidation des Reduktionsmittels durch Luftsauerstoff kann es auch zweckmäßig sein, EDTA, vorzugsweise in einer Menge von 1 bis 10 mmol/l, zuzusetzen. Neben der Solubilisierung/Reduktion hat die Solubilisierungsstufe auch einen Reinigungseffekt, da ein Großteil von Fremdproteinen nicht in Lösung geht.

Eine andere Ausführungsform der Erfindung beruht auf der Bildung der gemischten Disulfide von in Prokaryonten exprimierten biologisch aktiven Proteinen und Glutathion vor der Stufe der Reaktivierung. Zur Bildung der gemischten Disulfide werden die dialysierten, reduzierten, von Reduktionsmitteln gereinigten Proteine mit einer Denaturierungsmittel enthaltenden verdünnten Lösung von GSSG, z. B. 0,2 mol/l, inkubiert. Die Aktivierung erfolgt nach Abtrennung des Oxidationsmittels durch erneute Dialyse gegen den Guanidin/Hydrochlorid oder Harnstoff enthaltenden Puffer bei einem pH-Wert von 6 bis 10, einer GSH-Konzentration von 0,1 bis 5 mmol/l und mit einer nicht denaturierenden Konzentration eines Denaturierungsmittels über einen Zeitraum von 1 bis 300 Stunden.

In allen anderen Reaktionsschritten entspricht die Aktivierung des Proteins über die Bildung der gemischten Disulfide mit GSSG den Ausführungsformen für die Aktivierung von Proteinen des vorher genannten Teils der Erfindung. Bei dieser Ausführungsform liegt das pH-Optimum bei 6 bis 8 und die aktivierten Proteine sind im Renaturierungspuffer über längere Zeit stabil.

Erfindungsgemäß gelingt es, z.B. bei Antikörpern als zu reaktivierende Proteine, welche in Prokaryonten exprimiert wurden, mit einer Ausbeute von bis zu 30 % der Immunreaktivität zu reaktivieren. Dies entspricht in etwa der fachen Ausbeute der nach dem Stand der Technik bekannten Verfahren. Als Antikörper im Sinne der Erfindung sind auch deren übliche Fragmente zu verstehen.

Die nachfolgenden Beispiele erläutern in Verbindung mit den Abbildungen die Erfindung weiter. Wenn nicht anders angegeben, beziehen sich Prozentangaben auf Gew.-% und Temperaturangaben auf °C.

Es zeigen:
- Fig. 1: Die Sequenz von Plasmid pBT111 zur Expression der kappa-Kette von MAK 33 (Nukleotidposition 7 bis 663).
- Fig. 2: Die Sequenz von Plasmid p10169 zur Expression der Fd-Kette von MAK33 (Nukleotidposition 240 bis 917).

### Beispiel 1

### Expression von Antikörperfragmenten in E. coli

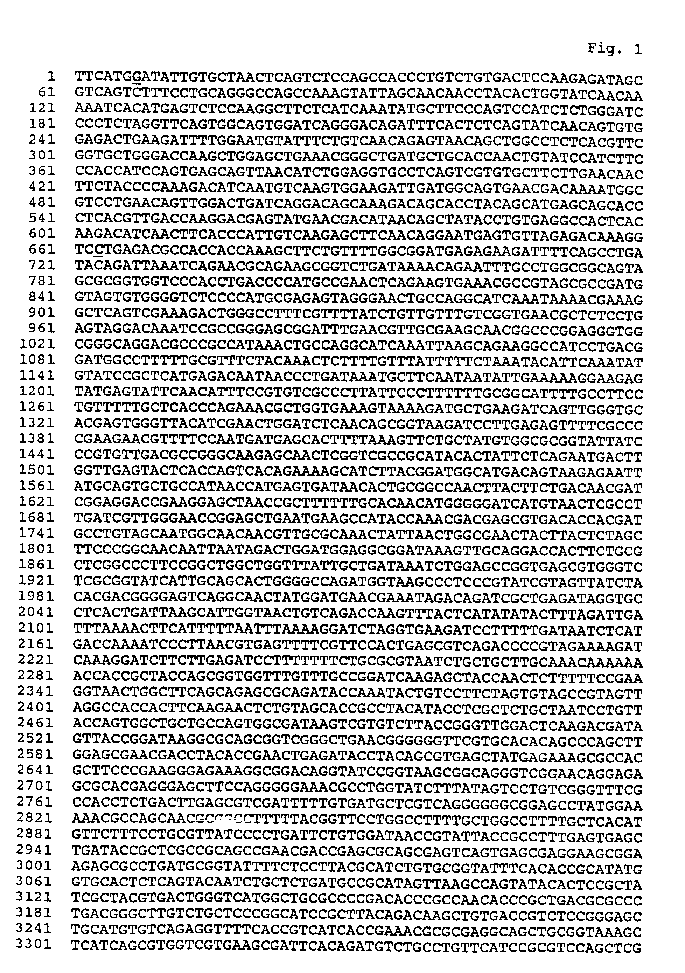

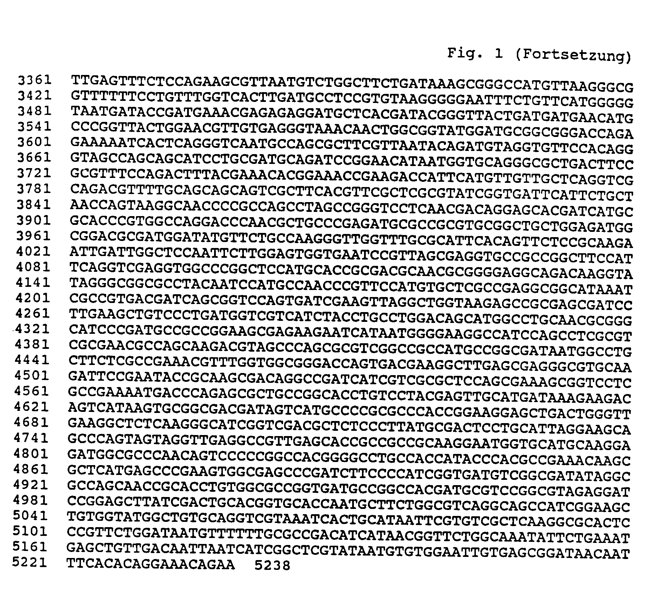

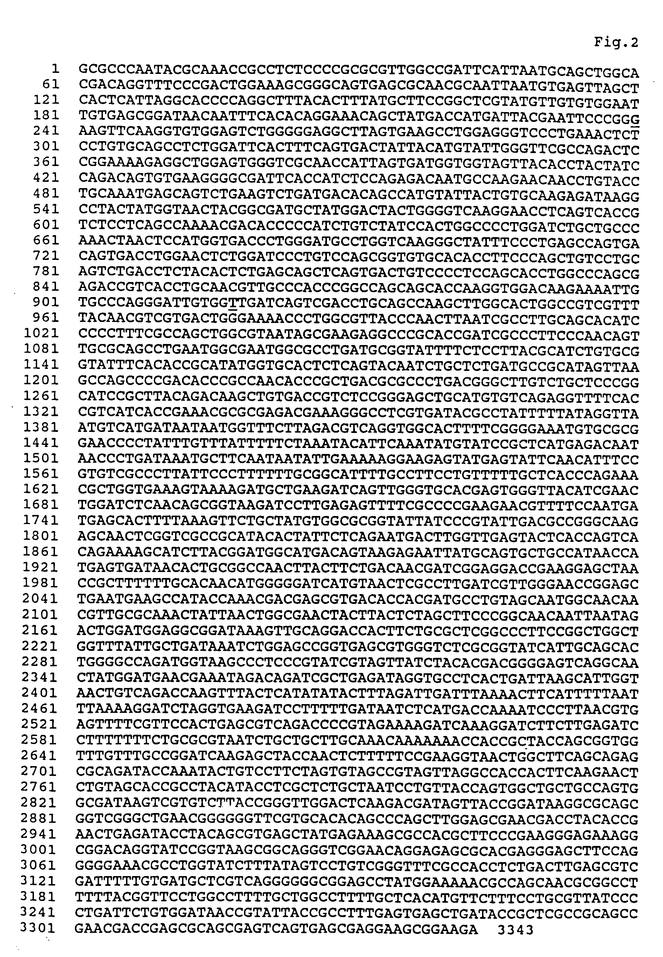

1.1 Konstruktion eines Plasmides zur Expression der MAK33 Kappa-Kette in E. coli
   Die Klonierung der Kappa-cDNA von MAK33 als PstI-Fragment in pBR322 wurde beschrieben (Buckel, P., et al. (1987), Gene 51, 13 - 19). Mit der Restriktionsendonuklease Mnl I wurde die cDNA unmittelbar 5′-benachbart des ersten Aminosäurecodons der reifen Kappa-Kette geschnitten und mit Hilfe eines Adapters (5′CATG3′ hybridisiert mit 5′CATGAATT3′) als EcoRI-PstI-Fragment in den ebenfalls mit EcoRI und PstI geschnittenen Vektor pKK223-3, DSM 3694P, (Brosius et al. (1981), Plasmid 6, 112-118) kloniert. Zur Verkürzung des 3′-untranslatierten Bereiches der cDNA wurde das resultierende Plasmid mit PstI geöffnet, mit Bal31 nukleolytisch verkürzt und anschließend das der Kappa-cDNA entsprechende EcoRI-Bal31-Fragment mit Hilfe eines HindIII-Linkers in den EcoRI HindIII geschnittenen Vektor pKK223-3 rückkloniert. Das resultierende Plasmid wird als pBT111 bezeichnet (Fig. 1 zeigt die Sequenz des Expressionsplasmides; Kappa von Nukleotidposition 7 bis 663).
1.2 Konstruktion eines Plasmides zur Expression des Fd-Fragments der gamma-Kette von MAK33 in E. coli
   Die Klonierung der MAK33 gamma-cDNA als PstI-Fragment in pBR322 wurde ebenfalls beschrieben (Buckel et al. 1987). Zur Expression wurde unmittelbar vor die erste Aminosäure der reifen gamma-Kette eine XmaI-Schnittstelle durch Oligonukleotid gerichtete Mutagenese eingeführt. Ein gamma-Fd-Fragment wurde durch Einführen eines Stop-Codons nach Aminosäure-Position 225 mit derselben Technik erzeugt, wobei zusätzlich eine BclI und eine SalI-Schnittstelle eingeführt wurden. Das resultierende XmaI-SalI-Fragment, welches für das gamma-Fd-Fragment kodiert, wurde in pUC8 (Vieira + Messing (1982) Gene 19, 259-268) kloniert. Fig. 2 zeigt die Sequenz des entstandenen Expressionsplasmides p10169 (gamma-Fd von Nukleotidposition 240 bis 917).
1.3 Expression der Antikörperketten in E. coli
   Die Expressionsplasmide pBT111 und p10169 wurden jeweils einzeln in E. coli (DSM 3689) transformiert, welcher außerdem ein Plasmid zur Expression des lac-Repressors (lacI^{q}) in trans enthielt. Die E. coli-Zellen wurden bis zu einer optischen Dichte OD₅₅₀ₙₘ = 0,5 auf LB-Medium gezüchtet, dann mit 1 g/l Isopropyl-β-D-thiogalaktosid (IPTG) induziert und weitere 4 Stunden bei 37°C inkubiert. Anschließend wurden die Zellen abzentrifugiert.
1.4 Präparation der "inclusion bodies"
   Hierzu wurden pro Immunglobulinkette ca. 25 g E. coli (vgl. Beispiel 1.3) Zellfeuchtmasse in 580 ml 0,1 mol/l Tris HCl, pH 6,5, 20 mmol/l EDTA aufgenommen und die Zellen mit dem Scherstab (Ultraturax) homogenisiert. Sodann wurden 0,25 mg/ml Lysozym zugegeben, 30 Minuten bei Raumtemperatur inkubiert und anschließend in 0,5 mol/l NaCl, 5 % v/v Triton-X-100 suspendiert und mit dem Scherstab (Ultraturax) homogenisiert und weitere 30 Minuten bei Raumtemperatur gerührt. Danach wurde eine Zentrifugation in einem Sorvall GSA-Rotor für 50 Minuten bei 4°C und 13.000 Upm durchgeführt. Die Pellets wurden in 300 ml 0,1 mol/l Tris^{·}HCl, pH 6,5, 20 mmol/l EDTA, 2,5 % v/v Triton-X-100 aufgenommen und homogenisiert. Sodann wurde eine weitere Zentrifugation für 30 Minuten bei 4°C und 13.000 Upm wiederum in einem Sorvall GSA-Rotor durchgeführt. Die Pellets wurden in 300 ml 0,1 mol/l Tris^{·}HCl, pH 6,5, 20 mmol/l EDTA, 0,5 % v/v Triton-X-100 aufgenommen und homogenisiert. Danach wurde noch zweimal je eine Zentrifugation für 30 Minuten bei 4°C und 13.000 Upm in einem Sorvall GSA-Rotor durchgeführt und jeweils danach die Pellets in 300 ml bzw. 250 ml 0,1 mol/l Tris HCl, pH 6,5, 20 mmol/l EDTA aufgenommen und homogenisiert.

### Beispiel 2

### Denaturierung der Antikörper

Lyophilisate von aus Hybridomazellinien (ECACC 88091404) erhaltenem Antikörper MAK 33 bzw. von Fab-Fragmenten davon (zur Produktion von Fab-Fragmenten, vgl. A. Johnstone und R. Thorpe in Immunochemistry in Practice, Blackwell Scientific Publications (1982), Seiten 52 bis 53), sowie die Pellets der "inclusion bodies"-Präparation nach Beispiel 1 wurden in 0,1 mol/l Tris^{·}HCl, pH 8,5; 6 mol/l Guanidin HCl; 2 mmol/l EDTA, 0,3 mol/l DTE 3 Stunden bei Raumtemperatur inkubiert. Die Proteinkonzentration betrug zwischen 4 und 6 mg/ml. Die Kettentrennung wurde mit SDS-PAGE unter nicht reduzierenden Bedingungen überprüft. Die vollständige Reduktion der Disulfidbrücken wurde durch die Bestimmung der freien SH-Gruppen nach G.L. Ellman (1959) Arch. Biochem. Biophys. 82, 70 bestätigt. Anschließend wurde der pH der Lösungen mit konzentrierter Salzsäure auf pH 3 eingestellt.

Die nachfolgend aufgeführten Beispiele zur Renaturierung wurden mit, wie in Beispiel 2 beschrieben, vollständig denaturiertem, reduziertem MAK 33 bzw. MAK 33 Fab oder den Antikörperketten aus den "inclusion bodies" in 1:100 Verdünnung in Reoxidationspuffer nach Dialyse gegen 6 mol/l Guanidinhydrochlorid, pH 2, durchgeführt. Die Renaturierungsansätze wurden bei 20°C thermostatisiert.

### Beispiel 3

### Renaturierung von MAK 33-Fab-Fragmenten.

Der Reoxidationspuffer enthielt 0,1 mol/l Tris HCl, pH 8,5, 0,5 mol/l L-Arginin, 2 mmol/l EDTA.

Die Proteinkonzentration betrug zwischen 30 und 60 µg/ml. Die Renaturierungsdauer betrug bis zu 200 Stunden. Die Reoxidation wurde mit dem konformationspezifischen ELISA-Testsystem (Beispiel 8) auf passive Immunreaktivität überprüft.

Die Tabellen 1A bis 1C zeigen die Ausbeute an aktivem MAK 33 Fab-Fragment in Abhängigkeit von der Variation:
1A der DTE-Konzentration bei konstanter GSSG-Konzentration (5 mmol/l GSSG);
1B der GSSG-Konzentration bei konstanter DTE-Konzentration (3 mmol/l DTE);
1C der GSSG-Konzentration bei konstanter GSH-Konzentration (1 mmol/l).

**Tabelle 1A**

| DTE (mmol/l) | Ausbeute (%) |
|---|---|
| 0 | 53 |
| 1 | 53 |
| 3 | 41 |
| 5 | 8 |
| 10 | 3 |

**Tabelle 1B**

| GSSG (mmol/l) | Ausbeute (%) |
|---|---|
| 0 | 18 |
| 1 | 15 |
| 3 | 23 |
| 5 | 32 |
| 10 | 37 |

**Tabelle 1C**

| GSSG (mmol/l) | Ausbeute (%) |
|---|---|
| 0 | 22 |
| 0,1 | 53 |
| 1 | 38 |
| 5 | 28 |

### Beispiel 4

Vollständig denaturierte, reduzierte MAK 33 Fab-Fragmente wurden gegen 6 mol/l Guanidinhydrochlorid, pH 2, dialysiert und anschließend in 0,1 mol/l Tris HCl, pH 8,5, 0,3 mol/l Guanidinhydrochlorid, 0,2 mol/l GSSG, 2 mmol/l GSH und 2 mmol/l EDTA bei einer Temperatur von 20°C und einer Renaturierungsdauer von ungefähr 200 Stunden reoxidiert. Tabelle 2 zeigt die Ausbeute an aktivem MAK 33 Fab in Abhängigkeit von der Variation der Proteinkonzentration bei der Renaturierung. Die Reoxidation wurde mit dem Elisa-Testsystem auf aktive Immunreaktivität (Beispiel 8) untersucht.

**Tabelle 2**

| Fab-Konzentration (µg/ml) | Ausbeute (%) |
|---|---|
| 5 | 13 |
| 10 | 9 |
| 20 | 4 |
| 30 | 3 |
| 70 | 1 |
| 130 | 0,5 |
| 660 | 0 |

### Beispiel 5

Vollständig denaturierte, reduzierte MAK 33 Antikörper wurden gegen 6 mol/l Guanidinhydrochlorid, pH 2, dialysiert und anschließend in 0,1 mol/l Tris HCl, pH 8,5, 0,5 mol/l L-Arginin, 2 mmol/l EDTA, 1 mmol/l GSH bei einer Temperatur von 20°C und einer Renaturierungsdauer von ungefähr 200 Stunden reoxidiert. Die Reoxidation wurde mit dem Elisa-Testsystem (Beispiel 8) auf aktive Immunreaktivität untersucht. Tabelle 3 zeigt die Ausbeute an aktivem Antikörper in Abhängigkeit der Variation der GSSG-Konzentration bei konstanter GSH-Konzentration (1 mmol/l).

**Tabelle 3**

| GSSG (mmol/l) | Ausbeute (%) |
|---|---|
| 0 | 2,0 |
| 0,1 | 3,5 |
| 0,5 | 4,7 |
| 1 | 4,8 |
| 2 | 4,2 |
| 4 | 3,9 |
| 6 | 3,4 |
| 10 | 2,7 |

### Beispiel 6

### Renaturierung nach Derivatisierung zum gemischten Disulfid

Die Denaturierung von MAK 33 bzw. MAK 33 Fab wurde, wie im Beispiel 2 beschrieben, durchgeführt. Sodann erfolgte eine Dialyse gegen 6 mol/l Guanidinhydrochlorid, pH 2, und daran anschließend die Derivatisierung mit 6 mol/l Guanidinhydrochlorid, 0,2 mol/l GSSG, 0,1 mol/l Tris HCl, pH 8,5 bei Raumtemperatur über einen Zeitraum von ungefähr 5 Stunden. Nach erneuter Dialyse gegen 6 mol/l Guanidinhydrochlorid, pH 2, wurde die Renaturierung in verschiedenen Varianten durchgeführt.
- Tabelle 4A: zeigt die Renaturierung von MAK 33 Fab in 0,1 mol/l Tris HCl, pH 7, 0,5 mol/l L-Arginin, 2 mmol/l EDTA, bei 20°C über etwa 200 Stunden. Die Renaturierung wurde mit dem konformationsspezifischen Elisa-Testsystem auf passive (Beispiel 8) Immunreaktivität getestet in Abhängigkeit von der GSH-Konzentration.
- Tabelle 4B: zeigt die Ausbeute an aktivem MAK 33 Fab in obigem Puffer mit 2 mmol/l GSH in Abhängigkeit vom pH des Puffers.
- Tabelle 5A: zeigt die Renaturierung von MAK 33 IgG in 0,1 mol/l Tris HCl, pH 7, 0,5 mol/l L-Arginin, 2 mmol/l EDTA bei einer Temperatur von 20°C über eine Renaturierungsdauer von ungefähr 200 Stunden in Abhängigkeit von der GSH-Konzentration. Die Renaturierung wurde mit dem konformationspezifischen Elisa-Testsystem auf passive Immunreaktivität (Beispiel 8) untersucht.
- Tabelle 5B: zeigt die Ausbeute an aktivem Antikörper in obigem Puffer mit 2 mmol/l GSH in Abhängigkeit vom pH-Wert.

**Tabelle 4A**

| GSH (mmol/l) | Ausbeute (%) |
|---|---|
| 0 | 5 |
| 0,1 | 48 |
| 0,5 | 48 |
| 1 | 41 |
| 2 | 36 |
| 3 | 31 |
| 4 | 27 |
| 7 | 24 |
| 10 | 23 |

**Tabelle 4B**

| pH | Ausbeute (%) |
|---|---|
| 7 | 40 |
| 8 | 39 |
| 9 | 30 |
| 10 | 19 |
| 11 | 1 |

**Tabelle 5A**

| GSH (mmol/l) | Ausbeute (%) |
|---|---|
| 0 | 0,3 |
| 0,1 | 3,0 |
| 0,2 | 4,0 |
| 0,5 | 3,8 |
| 1 | 3,1 |
| 2 | 2,5 |
| 3 | 2,0 |
| 6 | 1,4 |
| 10 | 1,4 |

**Tabelle 5B**

| pH | Ausbeute (%) |
|---|---|
| 6 | 3,9 |
| 7 | 3,8 |
| 8 | 2,9 |
| 9 | 1,5 |
| 10 | 0,8 |
| 11 | 0,6 |

### Beispiel 7

### Renaturierung von in E. coli exprimierten Antikörperketten.

Inclusion bodies der komplementären Einzelketten (Kappa und Fd, Beispiel 1) wurden wie im Beispiel 2 beschrieben, solubilisiert. Anschließend wurde gegen 6 mol/l Guanidinhydrochlorid, pH 2, dialysiert. Solubilisierung und Dialyse erfolgte separat für beide Ketten.

Zur Reoxidation wurden äquimolare Mengen der reduzierten Einzelketten gleichzeitig in einem 100fachen Volumen von 0,1 mol/l Tris-HCl, pH 8,5, 0,6 mol/l L-Arginin, 0,1 mmol/l GSSG, 1 mmol/l GSH und 2 mmol/l EDTA verdünnt. Die Renaturierung erfolgte durch Inkubation über 200 Stunden bei 20°C.

Die Ausbeute an nativem, biologisch aktivem Protein betrug 18%. Der Anteil an nativen Antikörpern wurde durch Messung der passiven Immunreaktivität (Beispiel 8) bestimmt.

### Beispiel 8

### Nachweis der passiven und aktiven Immunreaktivität sowie der Hemmaktivität.

8.1 Nachweis der passiven Immunreaktivität des monoklonalen Antikörpers gegen Human-CK-MM (Skelettmuskelisoenzym der humanen Creatinkinase)
   Unter passiver Immunreaktivität wird hier die Ausbildung nativstruktierter Epitope auf dem nativen bzw. renaturierten Antikörper verstanden. Die Detektion dieser Epitope erfolgt durch konformationsspezifische Anti-Maus-Antikörper, die aus einer anderen Tierart (hier: Schaf) gewonnen werden, in einem ELISA-Testsystem.
   Herstellung von konformationsspezifischen, polyklonalen Schaf-Anti-Maus-Fab-Antikörpern.
   Antiserum gegen polyklonales Maus-Immunglobulin wurde nach A. Johnstone und R. Thorpe, Immunochemistry in Practice, Blackwell Scientific Publications, Oxford, 1982, Seiten 27 bis 31 in Schafen hergestellt.
   Die IgG-Fraktion des Antiserums wurde nach Johnstone und Thorpe, Seiten 44 bis 46, über Ammonsulfatfraktionierung und DEAE-Ionenaustauscherchromatographie isoliert.
   500 mg dieser IgG-Fraktion wurden so oft an 20 ml eines Maus-Kappaketten-Spherosil Immunadsorbers (1,5 mg Maus-Kappakette/ml Spherosil) adsorbiert, bis durch Enzymimmuntest keine Anti-Maus-Kappa-Aktivität mehr nachweisbar war. Sodann wurde die IgG-Fraktion über eine Säule mit 20 ml einer Maus-Fab-Spherosil-Füllung (5 mg Maus-Fab/ml Spherosil) passiert und der spezifisch adsorbierte Anteil der IgG-Fraktion mit 0,2 mol/l Glycin, pH 2,8 eluiert. Nach Dialyse gegen 1 mmol/l Essigsäure wurde diese Teilfraktion lyophilisiert.
   Durch Immuntests wurde festgestellt: das so hergestellte IgG reagiert nicht mit freien M-Kappa-, M-gamma-, M-Fd-Ketten oder mit unvollständig gefalteten M-Kappa/M-Fd-Komplexen. Bindung findet statt mit M-Fab, M-IgG und vollständig gefalteten Komplexen von M-Kappa/M-Fd bzw. M-kappa/M-gamma.
   Renaturierte Antikörper und native Standardproben wurden mit konformationsspezifischen Anti-Maus-Fab-Antikörpern vorinkubiert. Je nach Anzahl nativ ausgebildeter Konformationsepitope wurden unterschiedlich viele Bindungsstellen des konformationsspezifischen Anti-Maus-Fab-Antikörpers gesättigt. Die Vorinkubationslösung wurde zusammen mit murinem Antikörper-Enzym-Konjugat (Antikörper-Peroxidase-Konjugat) in Teströhrchen gegeben, deren Wände mit Mäuseantikörpern beschichtet waren.
   Die ungesättigten konformationsspezifischen Anti-Maus-Antikörper aus Schaf verbrückten dann die wandgebundenen Mäuseantikörper mit den Mäuseantikörper-Enzym-Konjugaten, wobei die Menge an gebundenem Konjugat indirekt proportional der Menge an nativem bzw. renaturiertem Antikörper in der Vorinkubationslösung ist. Der spektroskopische Nachweis erfolgte nach Umsetzung des chromogenen Substrats ABTS® (2,2′-Azino-bis-(3-ethylbenzothiazolin-6-sulfonat)) durch das Antikörper-konjugierte Enzym durch Absorptionsmessung bei 405 nm (H.U. Bergmeyer, Meth. in Enzymol. 3rd Edition, Band 9, 15-37).
8.2 Nachweis der aktiven Immunreaktivität des monoklonalen Antikörpers
   MAK33 IgG erkennt spezifisch das Skelettmuskel-Isoenzym der humanen Creatinkinase (CK-MM; vgl. P. Buckel et al., Gene 51 (1987) 13-19).
   Unter aktiver Immunreaktivität wird hier die Reaktion des nativen bzw. renaturierten Antikörpers mit dem spezifischen Antigen (im vorliegenden Fall: Human-CK-MM) verstanden.
   In einem ELISA-Testsystem wurde diese Reaktion mit Hilfe Enzym-konjungierter, polyklonaler Anti-Maus-Antikörper über die Umsetzung des chromogenen Substrats ABTS kolorimetrisch quantitativ bestimmt.
8.3 Nachweis der Hemmaktivität der monoklonalen Antikörper
   MAK33 IgG erkennt ein Epitop, das nur auf den M Untereinheiten des skelettmuskelspezifischen Isoenzyms CK-MM ausgebildet wird. Durch Bindung an das Epitop wird die enzymatische Aktivität um 80% gehemmt (P. Buckel et al., 1987). Der CK-MM-Hemmtest ist somit ein sehr beweiskräftiger Test zum Nachweis der Rekonstitution von MAK33 nach vollständiger Denaturierung und Reduktion, da nur eine völlig renaturierte Antigenbindungsstelle das Enzym hemmen kann.
   Die Aktivität der Creatinkinase wurde mit Hilfe eines gekoppelten enzymatischen Tests der Firma Boehringer Mannheim bestimmt (H.U. Bergmeyer, Meth. of Enzym. Analysis, 3rd Edition, Band III, 510-540). Dabei setzt die Creatinkinase Creatinphosphat und ADP zu Creatin und ATP um. Um eine spektroskopisch nachweisbare Reaktion zu erhalten, wird das entstandene ATP von der Hexokinase zur Phosphorylierung von Glucose zu Glucose-6-Phosphat verbraucht. Glucose-6-Phosphat wird von der Glucose-6-Phosphatdehydrogenase unter Bildung von NADPH + H⁺ aus NADP⁺ zu Gluconat-6-Phosphat oxidiert. Aus der Extinktionsänderung pro Minute läßt sich die Aktivität der Creatinkinase berechnen.
   Aus einer Eichkurve mit nativem Antikörper gegen CK-MM läßt sich die Menge an nativem Material bestimmen, die der Hemmwirkung im Renaturierungsansatz entspricht. Der Anteil hemmaktiven Proteins, bezogen auf die Gesamtproteinmenge im Renaturierungsansatz, ergibt die prozentuale Ausbeute an hemmaktiven Antikörpern.
8.4 Vergleichende Bestimmung der Renaturierungsausbeute mittels passiver/aktiver Immunreaktivität und Hemmaktivität.
   Vollständig denaturierte, reduzierte MAK33-Fab-Fragmente wurden gegen 6 mol/l Guanidinhydrochlorid, pH 2, dialysiert und anschließend durch 1:100 Verdünnung in 0,1 mol/l Tris-HCl, pH 8,5, 0,5 mol/l L-Arginin, 0,2 mmol/l GSSG, 2 mmol/l GSH und 2 mmol/l EDTA bei einer Temperatur von 20°C und einer Renaturierungsdauer von etwa 200 Stunden reoxidiert.
   Aufgrund der relativ hohen Antikörperkonzentration, die bei der Messung der Hemmaktivität eingesetzt werden muß, wurde der Renaturierungsansatz konzentriert. Dazu wurde die Renaturierungslösung gegen 1 mmol/l Essigsäure dialysiert und anschließend lyophilisiert. Das Lyophilisat wurde in H₂O aufgenommen (H₂O : Renaturierungsvolumen = 1:200) und gegen 50 mmol/l Kaliumphosphat, 0,15 mol/l NaCl, pH 7,5 dialysiert.
   Tabelle 6 zeigt die Ausbeute an aktivem Fab-Fragment im Dialysat, bestimmt durch passive/aktive Immunreaktivität und Hemmaktivität.

**Tabelle 6**

| Testmethode | Ausbeute (%) |
|---|---|
| passive Immunreaktivität | 28 |
| aktive Immunreaktivität | 26 |
| Hemmaktivität | 25 |

### Beispiel 9

### Aktivierung von t-PA aus E.coli

Aus Zellfeuchtmasse von E. coli, DSM 3689, transformiert mit Plasmid pePa 133 (EP-A 0 242 835) werden wie in Beispiel 1.4 beschrieben "inclusion bodies" als Pellets präpariert.

Die Pellets der "inclusion body"-Präparation wurden in 0,1 mol/l Tris-HCl, pH 8,5; 6 mol/l Guanidin HCl; 2 mmol/l EDTA; 0,3 mol/l DTE 3 Stunden bei Raumtemperatur bei einer Proteinkonzentration zwischen 4 und 6 mg/ml inkubiert. Im Anschluß an die Solubilisierung wurde der pH-Wert der Lösung mit konzentrierter Salzsäure auf pH 3 eingestellt. Reduktionsmittel und Pufferbestandteile wurden durch Dialyse gegen 6 mol/l Guanidin-HCl, pH 2, 4°C abgetrennt.

### Renaturierung von t-PA

Renaturierung des so erhaltenen denaturierten, reduzierten Proteins erfolgte durch Verdünnung in 0,1 mol/l Tris-HCl, pH 10,5, 0,5 mol/l L-Arginin, 1 mmol/l EDTA und 1 mg/ml Rinderserumalbumin. Die Proteinkonzentration betrug zwischen 10 und 30 µg/ml, die Temperatur 20°C und die Renaturierungsdauer 24 h.

Die Reaktivierung wurde nach der Testvorschrift für tPA-Standard der Firma Boehringer Mannheim GmbH, Bestell-Nr. 1080954 bestimmt. Die Tabellen 7A und 7B zeigen die Ausbeute an aktivem tPA in Abhängigkeit von der Variation:
7 A der GSSG-Konzentration bei konstanter DTE Konzentration (2 mmol/l)
7 B der GSSG-Konzentration bei konstanter GSH-Konzentration (1 mmol/l)

**Tab. 7 A**

| GSSG (mmol/l) | Ausbeute (%) |
|---|---|
| 0 | 0 |
| 1 | 1 |
| 3 | 7 |
| 5 | 12 |
| 10 | 6 |

**Tab. 7 B**

| GSSG (mmol/l) | Ausbeute (%) |
|---|---|
| 0 | 6 |
| 0,1 | 14 |
| 1 | 10 |
| 5 | 1 |

### Beispiel 10

### Renaturierung von K2P (t-PA-Derivat, vgl. DE 39 03 581) nach Derivatisierung zum gemischten Disulfid

Wie in DE 39 03 581 beschrieben, werden Zellen von E. coli, DSM 2093, die mit den Plasmiden pA27fd und pUBS500 transformiert sind, gezüchtet und Zellfeuchtmasse gewonnen. Aus dieser Zellfeuchtmasse wird wie in Beispiel 9 beschrieben denaturiertes, reduziertes Protein gewonnen.

Die Lösung des denaturierten, reduzierten Proteins in 6 mol/l Guanidin-HCl, pH 2 wurde mit GSSG auf 0,1 mol/l und mit Tris auf 0,1 mol/l eingestellt. Anschließend wurde der pH-Wert mit NaOH auf pH 8,5 eingestellt. Nach 2 h Inkubation bei Raumtemperatur wurden Oxidationsmittel und Pufferbestandteile durch eine erneute Dialyse gegen 6 mol/l Guanidin-HCl, pH 2, 4°C abgetrennt.

Renaturierung des denaturierten, oxidierten Proteins ("gemischtes Disulfid") erfolgte durch Verdünnung in Renaturierungspuffer. Die Proteinkonzentration betrug zwischen 30 und 60 µg/ml, die Temperatur 20°C und die Renaturierungsdauer 12 h.
- Tab. 8 A: zeigt die Reaktivierung von K2P in 0,1 mol/l Tris-HCl, pH 8,5, 0,8 mol/l L-Arginin, 2 mmol/l EDTA in Abhängigkeit von GSH-Konzentration
- Tab. 8 B: zeigt die Ausbeute an aktivem K2P in obigem Puffer mit 0,5 mmol/l GSH in Abhängigkeit vom pH-Wert des Puffers.

**Tab. 8 A**

| GSH (mmol/l) | Ausbeute (%) |
|---|---|
| 0 | 0 |
| 0,1 | 14 |
| 0,5 | 26 |
| 1 | 20 |
| 2 | 10 |

**Tab. 8 B**

| pH | Ausbeute (%) |
|---|---|
| 7 | 6 |
| 8 | 15 |
| 9 | 23 |
| 10 | 7 |

## Patentansprüche

1. Verfahren zur Aktivierung von gentechnologisch hergestellten, in Prokaryonten exprimierten biologisch aktiven Proteinen nach Zellaufschluß durch Solubilisierung unter denaturierenden und reduzierenden Bedingungen und nachfolgende Reaktivierung unter oxidierenden und renaturierenden Bedingungen,
**dadurch gekennzeichnet,** daß man bei einer Proteinkonzentration von 1 bis 1000 µg/ml arbeitet und zwischen der Solubilisierung und der Reaktivierung eine Dialyse gegen einen Puffer mit einem pH-Wert zwischen 1 und 4, enthaltend 4 bis 8 mol/l Guanidinhydrochlorid oder 6 bis 10 mol/l Harnstoff, durchführt.

2. Verfahren nach Anspruch 1,
**dadurch gekennzeichnet,** daß der Dialyse-Puffer 5 bis 7 mol/l Guanidinhydrochlorid enthält.

3. Verfahren nach Anspruch 1,
**dadurch gekennzeichnet,** daß man die Reaktivierung bei einem pH-Wert von 9 bis 12, einer GSH-Konzentration von 0,1 bis 20 mmol/l, einer GSSG-Konzentration von 0,01 bis 3 mmol/l und mit einer nicht denaturierenden Konzentration eines Denaturierungsmittels über einen Zeitraum von 1 bis 300 Stunden durchführt.

4. Verfahren nach Anspruch 3,
**dadurch gekennzeichnet,** daß die GSH-Konzentration 0,2 bis 10 mmol/l und/oder die GSSG-Konzentration 0,05 bis 1 mmol/l beträgt.

5. Verfahren nach Anspruch 1 oder 2,
**dadurch gekennzeichnet,** daß man in der Stufe der Reaktivierung zuerst durch Zugabe von GSSG unter denaturierenden Bedingungen die Thiolgruppen der Proteine in die gemischten Disulfide von Protein und Glutathion überführt, erneut gegen den Guanidinhydrochlorid- oder Harnstoff enthaltenden Puffer dialysiert und dann bei einem pH-Wert von 6 bis 10, einer GSH-Konzentration von 0,1 bis 5 mmol/l und mit einer nicht denaturierenden Konzentration eines Denaturierungsmittels über einen Zeitraum von 1 bis 300 Stunden reaktiviert.

6. Verfahren nach Anspruch 3 bis 5,
**dadurch gekennzeichnet,** daß man in der Reaktivierungsstufe als Denaturierungsmittel Arginin, Guanidinhydrochlorid und/oder wenigstens eine Verbindung der allgemeinen Formel R₂-CO-NRR₁ (I), in der R und R₁ H oder Alkyl mit 1 bis 4 C-Atomen und R₂ H oder NRR₁ oder Alkyl mit 1 bis 3 C-Atomen bedeuten, verwendet.

7. Verfahren nach Anspruch 6, **dadurch gekennzeichnet,** daß die Konzentration an Arginin und/oder Guanidinhydrochlorid 0,1 bis 1,0 mol/l, insbesondere 0,25 bis 0,8 mol/l beträgt.

8. Verfahren nach Anspruch 6, **dadurch gekennzeichnet,** daß die Konzentration der Verbindung der allgemeinen Formel I 0,5 bis 4 mol/l, insbesondere 1 bis 3,5 mol/l beträgt.

9. Verfahren nach einem der vorhergehenden Ansprüche, **dadurch gekennzeichnet,** daß man in der Stufe der Reaktivierung in Gegenwart eines nicht proteolytisch wirksamen Proteins, insbesondere in Gegenwart von Rinderserumalbumin, arbeitet.

10. Verfahren nach einem der vorhergehenden Ansprüche, **dadurch gekennzeichnet,** daß man in der Stufe der Reaktivierung in Gegenwart von 1 bis 10 mmol/l EDTA arbeitet.

11. Verfahren nach einem der vorhergehenden Ansprüche, **dadurch gekennzeichnet,** daß man den Zellaufschluß mittels Ultraschall, Hochdruckdispersion oder Lysozym durchführt.

12. Verfahren nach Anspruch 11, **dadurch gekennzeichnet,** daß man den Aufschluß in einer verdünnten wäßrigen Pufferlösung, insbesondere in 0,1 mol/l Tris, bei einem neutralen bis schwach sauren pH-Wert durchführt.

13. Verfahren nach einem der vorhergehenden Ansprüche, **dadurch gekennzeichnet,** daß man nach dem Zellaufschluß die unlöslichen Bestandteile abtrennt.

14. Verfahren nach einem der vorhergehenden Ansprüche, **dadurch gekennzeichnet,** daß man in der Stufe der Solubilisierung bei einem alkalischen pH-Wert in Gegenwart eines Reduktionsmittels aus der Mercaptangruppe und in Gegenwart eines Denaturierungsmittels arbeitet.

15. Verfahren nach Anspruch 14, **dadurch gekennzeichnet,** daß man in Gegenwart von Guanidinhydrochlorid und/oder einer Verbindung der allgemeinen Formel I als Denaturierungsmittel arbeitet.

16. Verfahren nach Anspruch 15, **dadurch gekennzeichnet,** daß die Konzentration an Guanidinhydrochlorid 6 mol/l, die der Verbindung der allgemeinen Formel I 8 mol/l, beträgt.

17. Verfahren nach einem der Ansprüche 14 bis 16, **dadurch gekennzeichnet,** daß man in Gegenwart von DTE, β-Mercaptoethanol, Cystein oder GSH arbeitet.

18. Verfahren nach einem der vorhergehenden Ansprüche, **dadurch gekennzeichnet,** daß man Antikörper bzw. Antikörperfragmente reaktiviert.

19. Verfahren nach einem der Ansprüche 1 bis 17, **dadurch gekennzeichnet,** daß man t-PA oder ein t-PA-ähnlich wirkendes Protein reaktiviert.

## Claims

1. Process for the activation of gene-technologically produced, biologically-active proteins expressed in prokaryotes after cell digestion by solubilisation under denaturing and reducing conditions and subsequent reactivation under oxidising and renaturing conditions, characterised in that one works at a protein concentration of 1 to 1000 µg/ml and, between the solubilisation and the reactivation, carries out a dialysis against a buffer with a pH value between 1 and 4 containing 4 to 8 mol/l guanidine hydrochloride or 6 to 10 mol/l urea.

2. Process according to claim 1, characterised in that the dialysis buffer contains 5 to 7 mol/l guanidine hydrochloride.

3. Process according to claim 1, characterised in that one carries out the reactivation at a pH value of 9 to 12, a GSH concentration of 0.1 to 20 mmol/l, a GSSG concentration of 0.01 to 3 mmol/l and with a non-denaturing concentration of a denaturing agent over a period of time of 1 to 300 hours.

4. Process according to claim 3, characterised in that the GSH concentration amounts to 0.2 to 10 mmol/l and/or the GSSG concentration to 0.05 to 1 mmol/l.

5. Process according to claim 1 or 2, characterised in that, in the reactivation step, by addition of GSSG under denaturing conditions, one first converts the thiol groups of the proteins into the mixed disulphides of proteins and glutathione, again dialyses against the guanidine hydrochloride- or urea-containing buffer and then reactivates at a pH value of 6 to 10, a GSH concentration of 0.1 to 5 mmol/l and with a non-denaturising concentration of a denaturing agent over a period of time of 1 to 300 hours.

6. Process according to claim 3 to 5, characterised in that, in the reactivation step, as denaturing agent one uses arginine, guanidine hydrochloride and/or at least one compound of the general formula R₂-CO-NRR₁ (I), in which R and R₁ signify H or alkyl with 1 to 4 C-atoms and R₂ H or NRR₁ or an alkyl with 1 to 3 C-atoms.

7. Process according to claim 6, characterised in that the concentration of arginine and/or guanidine hydrochloride amounts to 0.1 to 1.0 mol/l, especially to 0.25 to 0.8 mol/l.

8. Process according to claim 6, characterised in that the concentration of the compound of the general formula I amounts to 0.5 to 4 mol/l, especially to 1 to 3.5 mol/l.

9. Process according to one of the preceding claims, characterised in that, in the reactivation step, one works in the presence of a non-proteolytically active protein, especially in the presence of bovine serum albumin.

10. Process according to one of the preceding claims, characterised in that, in the reactivation step, one works in the presence of 1 to 10 mmol/l EDTA,

11. Process according to one of the preceding claims, characterised in that one carries out the cell digestion by means of ultrasonics, high pressure dispersion or lysozyme,

12. Process according to claim 11, characterised in that one carries out the digestion in a dilute aqueous buffer solution, especially in 0.1 mol/l Tris, at a neutral to weakly acidic pH value.

13. Process according to one of the preceding claims, characterised in that, after the cell digestion, one separates off the insoluble components,

14. Process according to one of the preceding claims, characterised in that, in the solubilisation step, one works at an alkaline pH value in the presence of a reducing agent of the mercaptan group and in the presence of a denaturing agent.

15. Process according to claim 14, characterised in that one works in the presence of guanidine hydrochloride and/or of a compound of the general formula I as denaturing agent.

16. Process according to claim 15, characterised in that the concentration of guanidine hydrochloride amounts to 6 mol/l, that of the compound of the general formula I to 8 mol/l.

17. Process according to one of claims 14 to 16, characterised in that one works in the presence of DTE, β-mercaptoethanol, cysteine or GSH.

18. Process according to one of the preceding claims, characterised in that one reactivates antibodies or antibody fragments.

19. Process according to one of claims 1 to 17, characterised in that one reactivates tPA or a protein acting like tPA.

## Revendications

1. Procédé d'activation de protéines biologiquement actives, préparées par génie génétique et exprimées chez les procaryotes, après lyse des cellules par solubilisation dans des conditions dénaturantes et réductrices puis réactivation dans des conditions oxydantes et renaturantes, caractérisé en ce que l'on opère à une concentration en protéines de 1 à 1000 µg/ml et, entre la solubilisation et la réactivation, on réalise une dialyse contre un tampon de pH compris entre 1 et 4, contenant 4 à 8 mol/l de chlorhydrate de guanidine ou 6 à 10 mol/l d'urée.

2. Procédé selon la revendication 1, caractérisé en ce que le tampon de dialyse contient 5 à 7 mol/l de chlorhydrate de guanidine.

3. Procédé selon la revendication 1, caractérisé en ce que l'on réalise la réactivation pendant une durée de 1 à 300 heures à un pH de 9 à 12, une concentration en GSH de 0,1 à 20 mmol/l, une concentration en GSSG de 0,01 à 3 mmol/l et avec une concentration non dénaturante d'un agent dénaturant.

4. Procédé selon la revendication 3, caractérisé en ce que la concentration en GSH est de 0,2 à 10 mmol/l et/ou la concentration en GSSG est de 0,05 à 1 mmol/l.

5. Procédé selon la revendication 1 ou 2, caractérisé en ce que, dans l'étape de réactivation, on convertit d'abord les groupes thiol des protéines en les disulfures mixtes de protéine et de glutathion par addition de GSSG dans des conditions dénaturantes, on dialyse de nouveau contre le tampon contenant du chlorure de guanidine ou de l'urée puis on réactive pendant une durée de 1 à 300 heures à un pH de 6 à 10, une concentration en GSH de 0,1 à 5 mmol/l et avec une concentration non dénaturante d'un agent dénaturant.

6. Procédé selon les revendications 3 à 5, caractérisé en ce que, dans l'étape de réactivation, on utilise comme agent dénaturant l'arginine, le chlorhydrate de guanidine et/ou au moins un composé de formule générale R₂-CO-NRR₁ (I), dans laquelle R et R₁ représentent H ou alkyle de 1 à 4 atomes de carbone et R₂ représente H ou NRR₁ ou alkyle de 1 à 3 atomes de carbone.

7. Procédé selon la revendication 6, caractérisé en ce que la concentration en arginine et/ou en chlorhydrate de guanidine est de 0,1 à 1,0 mol/l, en particulier de 0,25 à 0,8 mol/l.

8. Procédé selon la revendication 6, caractérisé en ce que la concentration du composé de formule générale I est de 0,5 à 4 mol/l, en particulier de 1 à 3,5 mol/l.

9. Procédé selon l'une des revendications précédentes, caractérisé en ce que, dans l'étape de réactivation, on opère en présence d'une protéine non active du point de vue protéolytique, en particulier en présence de sérumalbumine bovine.

10. Procédé selon l'une des revendications précédentes, caractérisé en ce que, dans l'étape de réactivation, on opère en présence de 1 à 10 mmol/l de EDTA.

11. Procédé selon l'une des revendications précédentes, caractérisé en ce que l'on réalise la lyse des cellules au moyen d'ultrasons, d'une dispersion sous haute pression ou de lysozyme.

12. Procédé selon la revendication 11, caractérisé en ce que l'on réalise la lyse dans une solution tampon aqueuse diluée, en particulier dans du Tris à 0,1 mol/l, à un pH neutre à faiblement acide.

13. Procédé selon l'une des revendications précédentes, caractérisé en ce que, après la lyse des cellules, on sépare les constituants insolubles.

14. Procédé selon l'une des revendications précédentes, caractérisé en ce que, dans l'étape de solubilisation, on opère à pH alcalin en présence d'un réducteur du groupe des mercaptans et en présence d'un agent dénaturant.

15. Procédé selon la revendication 14, caractérisé en ce que l'on opère en présence de chlorhydrate de guanidine et/ou d'un composé de formule générale I en tant qu'agent dénaturant.

16. Procédé selon la revendication 15, caractérisé en ce que la concentration en chlorhydrate de guanidine est de 6 mol/l et la concentration du composé de formule générale I est de 8 mol/l.

17. Procédé selon l'une des revendications 14 à 16, caractérisé en ce que l'on opère en présence de DTE, de β-mercaptoéthanol, de cystéine ou de GSH.

18. Procédé selon l'une des revendications précédentes, caractérisé en ce que l'on réactive des anticorps ou des fragments d'anticorps.

19. Procédé selon l'une des revendications 1 à 17, caractérisé en ce que l'on réactive le t-PA ou une protéine agissant de manière analogue au t-PA.
